# EUROPEAN PATENT SPECIFICATION

(11) **EP 0 349 059 B1**
(45) Date of publication and mention of the grant of the patent: **08.01.1997**
(21) Application number: 89201631.2
(22) Date of filing: 21.06.1989
(51) Int. Cl.: C07H 13/06

(54) **Process for the synthesis of polyol fatty acid polyesters**
Verfahren zur Herstellung von Polyol-Fettsäure-Polyestern
Méthode pour la préparation des polysters de polyol et d'acide de gras

(30) Priority: 29.06.1988 GB 8815426
(43) Date of publication of application: 03.01.1990
(73) Proprietor: UNILEVER N.V., 3013 AL Rotterdam (NL); UNILEVER PLC, London EC4P 4BQ (GB)
(72) Inventor: Willemse, Gerardus Wilhelmus Marie, NL-3136 CP Vlaardingen (NL)
(74) Representative: Mulder, Cornelis Willem Reinier, Dr.

(56) References cited:
- EP-A- 0 132 941
- DE-A- 3 324 917

## Description

The present invention relates to a process for the synthesis of polyol fatty acid polyesters, in which a polyol and/or a fatty acid oligoester thereof, is esterified by reaction with fatty acid lower alkylester under substantially solvent-free conditions at elevated temperature and reduced pressure in the presence of a transesterification catalyst and, optionally an emulsifier. The invention in particular, although not exclusively, relates to a process for the synthesis of sugar fatty acid polyesters, such as sucrose fatty acid polyesters.

Polyol fatty acid polyesters, and in particular, the sugar fatty acid polyesters, such as e.g. the sucrose fatty acid poly-esters, are known as suitable low-calorie fat-replacers in edible products. Substantially indigestible for human beings they have physical and organoleptic properties very similar to triglyceride oils and fats conventionally used in edible products. In addition, polyol fatty acid polyesters are reported to have use as pharmaceutical agents e.g. in view of their ability to take up fat-soluble substances, such as in particular cholesterol, in the gastro-intestinal tract, and subsequently remove those substances from the human body.

In this specification the term "polyol" is intended to include any aliphatic or aromatic compound which comprises at least four free hydroxyl groups. Such polyols in particular include the group of sugar polyols, which comprises the sugars, i.e. the mono-, di and polysaccharides, the corresponding sugar alcohols and the derivatives thereof having at least four free hydroxyl groups. Examples of sugar polyols include glucose, mannose, galactose, xylose, fructose, sorbose, tagatose, ribulose, xylulose, maltose, lactose, cello-biose, raffinose, sucrose, erythritol, mannitol, lactitol, sorbitol, xylitol and alphamethylglucoside. A generally used sugar polyol is sucrose.

In this specification the term "polyol fatty acid polyester" is intended to include any such polyesters or mixtures thereof of which, on an average, at least 70 % of the polyol hydroxyl groups have been esterified with fatty acids.

In this specification the percentage of polyol hydroxyl groups ot the polyol fatty acid ester that on an average have been esterified. with fatty acids, will be referred to as the degree of conversion, the situation where all hydroxyl groups have been esterified corresponding to 100 % conversion.

In this specification the term "fatty acid" refers to C₈-C₂₄ fatty acids which may be saturated or unsaturated, and which may have straight or branched alkyl chains.

In general polyol fatty acid polyesters are synthesized by a process in which a polyol, such as a mono- or disaccharide, is reacted with a fatty acid lower alkylester, in general a fatty-acid methylester, in the presence of a transesterification catalyst, such as e.g. an alkali metal hydroxide or carbonate. In a first stage a polyol fatty acid mono- or oligoester is formed, which in a second stage is further reacted with excess fatty acid lower alkylester to form polyol fatty acid polyesters of the desired degree of esterification. Under certain conditions the two stages of the reaction can be combined into a single step.

Processes of this type are described in e.g. the US 3,963,699, US 4,517,360 (= EP-A-132941), and US 4,518,772.

To drive the transesterification reaction towards the desired high conversion to polyol fatty acid polyester it is necessary during the reaction to continuously remove the lower alcohol formed in the transfer of fatty acid residues from the lower alkyl fatty acid ester to the polyol or polyolester. In conventional processes removal of the lower alcohol from the reaction mixture is achieved by carrying out the reaction under conditions of reduced pressure.

It has now been found that the time required by the transesterification reaction to reach high degrees of conversion can be reduced and the reaction better controlled when during, in particular, the latter stage of the reaction the removal of the lower alcohol is increased by the use of a stripping agent.

Thus, the process according to the invention is as defined in claim 1.

Since catalyst efficiency is adversely influenced by long reaction times, the reduction of reaction times is of advantage not only for reasons of operating time and energy consumption, but also for reason of catalyst consumption.

Accordingly, the present invention provides a process for the synthesis of polyol fatty acid polyesters, in which a polyol and/or a fatty acid oligoester thereof, is esterified by reaction with fatty acid lower alkylester under substantially solvent-free conditions at elevated temperature in the presence of a transesterification catalyst and, optionally an emulsifier characterized in that at least during the final stage of the transesterification reaction the reaction mixture is submitted to the action of a stripping agent suitable to accelerate the removal of the lower alkyl alcohol formed in said reaction.

The process in accordance with the invention is preferably applied to achieve high degrees of conversion, such as degrees of conversion of 85 % and above, and in particular 95 % or even 97 % and above, without the need of excessive transesterification times.

Any stripping agent which under the conditions of the reaction (described hereunder in more detail) accelerates the removal of the lower alkyl alcohol and which does not adversely interferes with the transesterification reaction, can suitably be employed. Suitable such stripping agents include inert gases, such as nitrogen, and volatile (under reaction conditions) organic compounds having low or no chemical activity under reaction conditions. A particularly preferred stripping agent is hexane.

The appropriate flow of stripping agent through the reaction mixture is dependent upon the reaction conditions and the dimensions of the equipment, in particular of the reaction vessel. In general, and particularly during the final stage of the transesterification reaction, the flow of stripping agent lies within the range of 100 to 2500 litres of stripping agent per hour and per kg of reaction mixture.

The amount of stripping agent is expressed as litres under the pressure and temperature conditions of the reaction mixture at the moment of stripping. Expressed in this way, the flow ranges are independent of temperature and pressure.

Preferably, a flow of stripping agent of more than 200, in particular of between 250 and 1500 litres/hour/kg of reaction mixture is used, the range of 300 to 1000, or even 500 to 1000 litres/hour/kg being preferred most.

Suitable contact between the stripping agent and the reaction mixture is normally established due to the whirling action caused by the stripping agent flowing through the reaction mixture. However, it may be desirable to apply further agitation by way of appropriate strirrer means.

Preferably, after leaving the reaction mixture the stripping agent is first, at least partly, separated from the lower alkyl alcohol, and subsequently recirculated to the reaction mixture.

In a further preferred aspect of the invention a sufficiently volatile lower alkyl fatty ester is used as stripping agent. Suitable such volatile lower alkyl fatty acid esters are lower alkyl esters, in particular methyl esters, of fatty acids having a fatty acid chain length of less than 15 carbon atoms, in particular, of 6 to 12 carbon atoms, chain lengths of 10 to 12 carbon atoms being preferred.

The sufficiently volatile lower alkyl fatty acid ester used as stripping agent may be introduced as such during the reaction, or as part of the blend of lower alkyl fatty acid ester used as active reactant in the transesterification reaction. Suitable such blends have fatty acid residues derived from vegetable oils such as in particular the lauric fats, e.g. palm kernel oil and coconut oil, comprising a relatively large proportion of the appropriate short chain fatty acids.

The amount of lower alkyl fatty acid ester stripping agent used during the reaction, calculated as percentage by weight of the total amount of lower alkyl fatty acid ester, should be at least 5 %, an amount within the range of 10 to 20 % being preferred.

The transesterification reaction is suitably carried out at a temperature which normally lies within the range of from 100 to 180°C. Preferably temperatures are applied within the range of from 110 to 160°C, the range of from 120 to 150°C being preferred most.

It has been found of advantage to control the partial vapour pressure of the lower alkyl alcohol such that in an initial stage (a) the polyol is esterified to a degree of conversion within the range of 10 to 50 %, preferably within the range of 10 to 30 %, substantially without leaving non-participating polyol (i.e. leaving less than about 20 %, calculated by weight of the initial polyol, of any polyol material that will remain unreacted throughout the transesterification reaction), and that in a subsequent stage the reaction mixture resulting from stage (a) is further esterified in accordance'with the process of the present invention.

Preferably, during the final part of stage (a) the partial vapour pressure of the lower alkyl alcohol is controlled to a level of within 3 kPa (30 mbar), most preferably within 1.5 kPa (15 mbar) from the equilibrium vapour pressure of the lower alkyl alcohol corresponding to a degree of conversion within the range of from 10 to 30%. Accordingly, the partial vapour pressure of the lower alkyl alcohol is suitably controlled to a level within the range of from 6 to 15 kPa (60 to 150 mbar), preferably 9 to 12.5 kPa (90 to 125 mbar) during the final part of stage (a).

During the first part of stage (a), in order to initiate the transesterification reaction quickly, it is of advantage to apply a pressure above the reaction mixture as low as technically and economically feasible, such as e.g. below 2.5 kPa (25 mbar), or even below 1 kPa (10 mbar). Subsequently, the pressure can be allowed to increase to the preferred levels described hereinbefore due to progressive formation of lower alkyl alcohol.

Subsequent to stage (a) the pressure is reduced to a level of below 5 kPa (50 mbar), and preferably to a level of below 2.5 kPa (25 mbar), such as below 1.5 kPa (15 mbar), or even below 0.5 kPa (5 mbar) to remove the lower alkyl alcohol formed from the fatty acid lower alkylester as a result of the transesterification. It is in particular during the final part of this stage of the reaction that the reaction mixture is submitted to the action of the stripping agent in accordance with the invention to further assist in the removal of the lower alkyl alcohol. The action of the stripping agent is preferably started when the transesterification reaction has progressed to a degree of conversion of between 60 and 95 %, and most preferably when the degree of conversion has reached a level of between 80 and 95 %.

It is preferred to apply agitation to the reaction mixture, throughout the reaction e.g. by way of stirring means in the reaction vessel.

In general the reactants used as the starting mixture of the transesterification reaction in accordance with the process of the present invention comprise a polyol, optionally in combination with a fatty acid oligoester thereof, a fatty acid lower alkylester, a basic transesterification catalyst, a fatty acid alkali metal soap, and solvent, such as water and/or alcohols.

The polyol may be any of those as defined hereinbefore, or a mixture thereof. Preferred polyol starting materials are the sugar polyols, and in particular, sucrose.

Suitable fatty acid lower alkylesters are fatty acid esters of the group of lower alcohols including mono-, di- and triols. In particular, the ester is derived from the C₁-C₅ mono-alcohols, preferably methanol. The fatty acids can be any of those as defined hereinbefore, the selection of which is dependent of the specific polyol fatty acid esters desired.

The amount of fatty acid lower alkylester is dependent of the desired degree of conversion. In general an excess amount of the fatty acid lower alkylester is used. For instance, in the synthesis of 100% converted sucrose good results are obtained when a molar ratio of fatty acid lower alkylester : sucrose is used within the range of from 10:1 to 20:1.

Suitable transesterification catalysts include the group consisting of alkali metals and alkaline earth metals, and their alkoxides, bicarbonates, carbonates, hydrides, hydroxides, and their alloys. KOH has been found to be particularly suitable, but also NaOH and the corresponding carbonates, and bicarbonates of potassium or sodium can be advantageously used. Although one might argue that the above reagents are not the catalysts themselves, but are reagents forming the catalyst, in this specification as is done in the literature relating to similar processes, this group will be referred to as catalysts.

The catalyst is used in an amount corresponding to a molar ratio of catalyst : polyol of at least 0.01:1, preferably of 0.05:1 to 1:1.

In general an emulsifier will be used in order to improve contact between the polyol, the catalyst and the fatty acid lower alkyl ester. Many types of alkali-resistant emulsifiers can suitably be used, such as edible emulsifiers including phosphatides, such as lecithin, and detergents, such as soaps, alkali metal alkyl sulphates, and sugar oligoesters of fatty acids. It is preferred to use alkali metal soaps derived from any of the fatty acids as defined hereinbefore. The alkali metal soap may be introduced as such, but preferably the soap is prepared in-situ, for example by partial saponification of the fatty acid lower alkyl esters as used in the trans-esterification reaction or by neutralisation of any fatty acids added. At in-situ preparation of the fatty acid soap it is preferred to use a solvent in which the alkaline substance used for the saponification or neutralization dissolves so as to improve contact during the saponification or neutralization reaction.

Conversion rates of polyol to polyol fatty acid ester are advantageously affected when a fatty acid soap is used preferably comprising at least 15 %, or even at least 75 % of short chain fatty acid soap, having a fatty acid chain length of less than 15 carbon atoms, in particular 6 to 12 carbon atoms.

A solvent is used to improve addition and mixing of the various reactants. Suitable solvents include water and/or lower alcohols, such as the C₁-C₅ alcohols.

Advantagously, intimate mixing of the reactants amd simultaneous solvent removal is achieved by combining the reactants into a mixture, and passing this mixture through a spraying nozzle into a drying chamber. Intimate mixing occurs due to the dissipation of energy on passing through the spraying nozzle. Evaporation of the solvent occurs in the drying chamber, the resulting vapour continuously being removed from the drying chamber by suitable reduced pressure or gas flow conditions. Adequate solvent evaporation may be established by a variety of per se conventional techniques, including the application reduced pressure and/or elevated temperature conditions, or the us optionally heated, co-current, counter-current or mixed-current inert gas flows. In a batch-wise operation the drying chamber is also suitably used as reaction vessel for the transesterification reaction. In a continuous or semi-continuous operation the drying chamber and reaction vessel preferably are separate.

It may be of further advantage to pre-mix the reactants before passing through the spraying nozzle by an alternative agitation step for example employing a dynamic or static mixer, or flow restriction in the feed line to the spraying nozzle.

It is preferred to prepare the initial mixture of reactants by way of a two-step process.

In a first step the polyol or the fatty acid oligoester thereof is mixed with the catalyst in a liquid system so as to form the corresponding polyol anion. The formation of the actual polyol anion may be immediate or only be realized under substantially solvent-free conditions. Preferably, the liquid mixture further comprises a solvent used to improve the contact between the polyol or the oligoester thereof and the catalyst. Suitable such solvents include water, lower alcohols and mixtures thereof. In particular water is a suitable solvent if potassium hydroxide or sodium hydroxide is used as the transesterification catalyst.

In the second step the liquid system is combined with the fatty acid lower alkyl ester. The fatty acid soap may be introduced into the mixture, either separately, or as part of the liquid system of step 1, or in combination with the fatty acid lower alkyl ester.

Suitably, the starting mixture of reactants resulting after step 2 is subsequently agitated and solvent is removed therefrom by a spray-drying step as described hereinbefore.

It is even more preferred first to prepare a mixture comprising the fatty acid lower alkylester and the soap emulsifier, which mixture is spray-dried before it is combined with further reactants, in particular the liquid system of step 1. By this route which accordingly comprises two spray-drying steps, optimal mixing of and solvent removal from the starting mixture of reactants is achieved by a process that can easily be applied on a technical scale and by continuous or semi-continuous operation.

If so desired a supplementary amount of polyol may be introduced to the starting mixture of reactants before starting the transesterification reaction.

The invention will now be further illustrated with reference to following example.

Using a two-step spray-drying process first a reaction mixture was prepared having the following composition:

| ingredients | % by weight |
|---|---|
| methylesters of soybean oil fatty acids | 90 |
| potassium soap of coconut fatty acids | 3 |
| sucrose (25 % as potassium sucrate) | 7 |
| | |
| OH-value | 90 |

In a 200 l reaction vessel fitted with stirrer, condensor and vacuum means 150 kg of this reaction mixture was caused to react to the partial sucrose ester (degree of conversion of about 20 %; OH-value of 70) in 3 hours at 135°C and 10 kPa (100 mbar) pressure. Subsequently, the pressure was reduced to below 0.5 kPa (5 mbar) (maintaining the temperature at 135°C), the degree of conversion increasing to approximately 90% (OH-value of 9). During the final stage of the transesterification reaction at 135°C and at about 1.5 kPa (15 mbar) a flow of hexane vapour was introduced into the reaction mixture in an amount of about 120 m³/hour (corresponding to 800 litres per hour per kg of reaction mixture). After one hour of stripping (t₁) with hexane vapour the degree of conversion has increased to approximately 96 % (OH-value of 4.5) and after two hours of stripping (t₂) to approximately 98 % (OH-value of 2.3).

In a comparative experiment equal amounts of ingredients were used, and reacted under the same process conditions and during the same process times, but without applying a stripping agent at the final stage of the reaction. At time t₁ the degree of conversion now was 95 % (OH-value of 5.5) and at t₂ the degree of conversion had reached 96 %.

These comparative experiments illustrate the usefulness of the process of the invention particularly when very high degrees of conversion are aimed at, such as degrees of conversion of over 95 %.

## Claims

1. Process for the synthesis of polyol fatty acid polyesters, in which a polyol and/or a fatty acid oligoester thereof is esterified by reaction with fatty acid ester of a lower alkyl alcohol under substantially solvent-free conditions at elevated temperature in the presence of a transesterification catalyst and optionally an emulsifier, characterised in that when the transesterification reaction has progressed to a degree of conversion of between 60 and 95%, the reaction mixture is submitted to the action of a stripping agent suitable for accelerating the removal of the lower alkyl alcohol formed in said reaction, said stripping agent being added to the reaction mixture at a flow within the range of 100 to 2500 litres/hour, expressed under the pressure and temperature conditions of the reaction, per kg of reaction mixture, wherein, if the stripping agent is a fatty acid lower alkyl ester, its fatty acid has a chain length of less than 15 carbon atoms.

2. Process according to claim 1, in which said stripping agent is hexane.

3. Process according to claim 1 or 2, in which said flow is within the range of 300 to 1000 litres/hour per kg of reaction mixture.

4. Process according to claim 1, in which said stripping agent is a lower alkyl fatty acid ester which is volatile under the reaction conditions.

5. Process according to claim 4, in which said fatty acid residues have a chain length of from 6 to 12 carbon atoms.

6. Process according to claim 4 or 5, in which the amount of lower alkyl fatty acid ester used as a stripping agent is 10 to 20% by weight of the total amount of lower alkyl fatty acid esters.

7. Process according to any one of the preceding claims, in which said stripping agent is recirculated.

8. Process according to any one of the preceding claims, in which during the final stage said reaction is carried out at a pressure of below 5 kPa (50 mbar).

9. Process according to any one of the preceding claims, in which the action of the stripping agent is started when the transesterification reaction has progressed to a degree of conversion of between 80 and 95%.

## Patentansprüche

1. Verfahren zur Herstellung von Polyol-Fettsäure-Polyestern, bei dem ein Polyol und/oder ein Fettsäure-Oligoester davon durch Reaktion mit Fettsäureester eines niederen Alkylalkohols unter im wesentlichen lösungsmittelfreien Bedingungen bei erhöhter Temperatur in Gegenwart eines Umesterungskatalysators und fakultativ eines Emulgators verestert wird, dadurch gekennzeichnet, daß dann, wenn die Umesterungsreaktion bis zu einem Umwandlungsgrad zwischen 60 und 95 % fortgeschritten ist, die Reaktionsmischung der Einwirkung eines Strippmittels unterworfen wird, das geeignet ist, die Entfernung des in der Reaktion gebildeten niederen Alkylalkohols zu beschleunigen, wobei das Strippmittel zur Reaktionsmischung bei einem Fluß innerhalb des Bereiches von 100 bis 2500 l/h, ausgedrückt unter den Druck- und Temperaturbedingungen der Reaktion, pro kg Reaktionsmischung zugefügt wird, worin, falls das Strippmittel ein Fettsäure-Niederalkylester ist, seine Fettsäure eine Kettenlänge von weniger als 15 C-Atomen aufweist.

2. Verfahren nach Anspruch 1, bei welchem das Strippmittel Hexan ist.

3. Verfahren nach Anspruch 1 oder 2, bei welchem der Fluß innerhalb des Bereiches von 300 bis 1000 l/h pro kg Reaktionsmischung liegt.

4. Verfahren nach Anspruch 1, bei welchem das Strippmittel ein Niederalkyl-Fettsäureester ist, der unter den Reaktionsbedingungen flüchtig ist.

5. Verfahren nach Anspruch 4, bei welchem die Fettsäurereste eine Kettenlänge von 6 bis 12 C-Atomen aufweisen.

6. Verfahren nach Anspruch 4 oder 5, bei welchem die Menge des als Strippmittel verwendeten Niederalkyl-Fettsäureesters 10 bis 20 Gew.-% der Gesamtmenge des Niederalkyl-Fettsäureesters beträgt.

7. Verfahren nach irgendeinem der vorhergehenden Ansprüche, bei welchem das Strippmittel rezirkuliert wird.

8. Verfahren nach irgendeinem der vorhergehenden Ansprüche, bei welchem die Reaktion während der Endstufe bei einem Druck von weniger als 5 kPa (50 mbar) durchgeführt wird.

9. Verfahren nach irgendeinem der vorhergehenden Ansprüche, bei welchem die Einwirkung des Strippmittels begonnen wird, wenn die Umesterungsreaktion bis zu einem Umwandlungsgrad zwischen 80 und 95 % fortgeschritten ist.

## Revendications

1. Procédé de synthèse de polyesters d'acides gras/polyol, dans lequel on estérifie un polyol et/ou son oligoester d'acide gras par réaction avec un ester d'acide gras d'un alcool alkylique inférieur dans des conditions sensiblement exemptes de solvant à température élevée en présence d'un catalyseur de transestérification et facultativement un émulsifiant, caractérisé en ce que, quand la réaction de transestérification a progressé à un degré de conversion entre 60 et 95%, le mélange de réaction est soumis à l'action d'un agent de stripage approprié pour accélérer l'enlèvement de l'alcool alkylique inférieur formé dans ladite réaction, ledit agent de stripage étant ajouté au mélange de réaction à un débit dans la gamme de 100 à 2500 litres/h, exprimé dans les conditions de pression et de température de la réaction, par kg du mélange de réaction, dans lequel, si l'agent de stripage est un alkylester inférieur d'acide gras, son acide gras a une longueur de chaîne de moins de 15 atomes de carbone.

2. Procédé selon la revendication 1, dans lequel ledit agent de stripage est l'hexane.

3. Procédé selon la revendication 1 ou 2, dans lequel ledit débit est dans la gamme des 300 à 1000 litres/h par kg de mélange de réaction.

4. Procédé selon la revendication 1, dans lequel ledit agent de stripage est un alkylester d'acide gras inférieur qui est volatil dans les conditions de réaction.

5. Procédé selon la revendication 4, dans lequel lesdits résidus d'acides gras ont une longueur de chaîne de 6 à 12 atomes de carbone.

6. Procédé selon la revendication 4 ou 5, dans lequel la quantité d'alkylester d'acide gras inférieur utilisé comme agent de stripage est 10 à 20% en poids de la quantité totale d'alkylesters d'acides gras inférieurs.

7. Procédé selon l'une quelconque des revendications précédentes, dans lequel ledit agent de stripage est remis en circulation.

8. Procédé selon l'une quelconque des revendications précédentes, dans lequel pendant le stade final, on effectue ladite réaction à une pression en dessous de 5 kPa (50 mbar).

9. Procédé selon l'une quelconque des revendications précédentes, dans lequel l'action de l'agent de stripage est débutée quand la réaction de transestérification a progressé à un degré de conversion entre 80 et 95%.
